# EUROPEAN PATENT APPLICATION

(11) **EP 4 792 810 A2**
(43) Date of publication of application: **19.08.2026**
(21) Application number: 26158318.1
(22) Date of filing: 12.02.2026
(51) Int. Cl.: A61K 31/28, A61K 31/4045, A61K 31/385, A61K 38/05, A61K 45/06, A23L 33/15, A23L 33/16, A61P 1/02, A61P 43/00

(54) **USE OF A COMBINATION COMPRISING MELATONIN FOR THE REJUVENATION OF THE WHOLE BODY AND SUBSEQUENT AGING SLOWDOWN**

(30) Priority: 12.02.2025 LU 103537
(71) Applicant: Bogrash, Philip, 3061852 Or Akiva (IL)
(72) Inventor: Bogrash, Philip, 3061852 Or Akiva (IL)
(74) Representative: Valet Patent Services Limited

(57) **Abstract**

Supplement and vitamin treatment for initial biological age reduction followed by drastic slowdown of aging, as well as periodontitis and caries prevention. The proposed vitamin and supplement based regimen comprises the daily taking of a chromium supplement and melatonin, although a combination of melatonin and alpha-lipoic acid is another possibility. Also optionally: for men daily taking of vitamin E and optionally other testosterone boosting supplement(s) and for women optionally taking a peptide-based hormone restoration supplement or melatonin alone (prior to chromium supplementation); which has the ability to restore the hormone production and even restore menstrual cycles for menopausal and perimenopausal women. The proposed regimen could be used by individuals independently, but is primarily intended for large scale governmental programs to rejuvenate the targeted population, thereby improving at a very low cost the population's health and producing a population increase due to postponed mortality and extended fertility period.

## Description

### 1. Field of the Invention

This invention relates to treatments for the rejuvenation of the whole body with subsequent aging slowdown and the same treatment is usable for caries and periodontitis prevention.

### 2. Description of the Prior Art

At the present time there is a very large number of products referred to as anti-aging such as cosmetic creams, ointments etc that merely help maintain the appearance of younger skin by means of peeling the upper layer of skin, filling the wrinkles, artificially tightening the skin by various facelift techniques or actually healing or mitigating the age and sunlight exposure induced skin damage such as by rubbing in collagen, vitamins etc into the skin. All of these means and methods have usually very limited results and only aim to create and preserve the appearance of the younger skin. There are other genuinely effective anti-aging or aging reduction methods aiming to improve not just the skin condition topically, but to rejuvenate the whole body by means of reversing the accumulated damages in the cells, especially cell mitochondrias, such as by using pterostylbene or resveratrol/trans-resveratrol in combination with nicotinamide mononucleotide (aka NMN), nicotinamide riboside (NR) and other comparable compounds related to vitamin B3 such as niacin, niacinamide which produce surtuins activation and restore cell metabolism at the mitochondrial level. The use of these supplements is described in patent application WO/2015/069860 and the use of NMN analogs and other vitamin B3 derivatives is described in patent application WO/2018/12040, whereas the use of resveratrol is described in CN101371827. These supplements can produce noticeable overall body rejuvenation which most visibly manifests itself in the improved skin condition, especially for the older persons where the positive changes are most pronounced. However they sometimes work well only when methyl-donor supplements are used (for example betaine), they do not work for everybody and sometimes stop working for a person for whom they used to work and commonly over time their effectiveness diminishes. Secondly a recent study (1), has found out that metabolites of niacin produce about 60% increase in incidence of atherosclerosis and cardio-vascular events. As pertinent metabolites produced in the body (2PY and 4PY) are the same (2) - the study's results are fully applicable to NMN and nicotinamide riboside (NR). In order to protect blood vessels from the above described damage additional prescription medication is used. For example Prof. David Sinclair who is the leading researcher and a major proponent of the above described rejuvenation treatment, has his own current supplement regimen published every year (3), includes in that published regimen the diabetes prescription drug metformin which is known to be *inter alia* used to treat atherosclerosis and for prevention of cardio-vascular events. Furthermore pterostilbene, better quality resveratrol/trans-resveratrol, nicotinamide riboside and nicotinamide mononucleotide are relatively highly expensive and therefore in terms of cost are not well suited for mass usage to prevent productive population's aging and relative numerical decline in the context of their lower birth rates. The other class of compounds that can be used for this purpose are known geroprotectors such as; carnosine, melatonin, delta sleep inducing peptide, spermidine, ergothioneine. alpha-ketoglutaric acid, rapamycin etc; they can produce a rejuvenating effect on the whole body which would most visibly be manifested in the improved skin condition. The use of geroprotector melatonin is featured in EP1991222. While these compounds substantially reverse and subsequently slow the aging process most visibly resulting in the youthening of the skin condition, the aging process although slower still remains not slow enough. A different approach has been known in the art for a long time and is based on hormone levels maintenance therapies to assure that male or female hormone levels respectively are maintained at levels corresponding to a younger person's levels. This tends to significantly slow down the aging process as it relates to the skin condition and as concerns any decline in muscle mass, cognitive ability and overall body's physiological condition. This invention aims to use a geroprotector melatonin (4) based therapy in combination with supplements which have long been used to improve carbohydrate metabolism, but have no rejuvenation effect by themselves; such as chrome supplements (5) or alpha lipoic acid (6) to produce a synergistic effect making it possible, if chromium supplements are used, depending on the dosage; possibly within a few weeks to achieve rejuvenation far exceeding what can be achieved by the use of hormonal or B3 derivatives in combination with resveratrol/pterostylbene or by the use of geroprotector-based therapies. For example it has been observed to visibly reset the biological ages for persons in their fifties typically by 15 - 20 years, in conjunction with large weight loss for those significantly overweight, if 3 to 5 mg melatonin daily dosage is used in conjunction with 400 - 500 mcg of a chromium supplement, such as for example chromium picolinate (Cr(Pic)3), within one to two weeks of treatment and then to slow aging down to imperceptible levels. However such drastic rejuvenation and body transformation within a very short time isn't advisable and can be extended over two to three months by means of reducing melatonin dosage to 1.5 mg per day as used by the author of this invention (hereinafter the author) . For women of about 50 years of age or older their levels of gender specific hormones usually >>>>won't be sufficient because of menopause related hormone levels drastic decline. In some cases for men especially those over 50 testosterone levels are also insufficient. Therefore taking supplements which induce secretion of said gender hormones is the third optional part of the treatment regimen of the present invention, as sustaining of reduced biological age achieved by means of rejuvenation with the melatonin and chromium supplements is most achievable if the blood levels of gender specific hormones are maintained at levels within the normal range corresponding to biological age produced by said rejuvenation. The importance of maintaining the levels of said hormones at levels corresponding to younger biological age is especially pronounced, as concerns the muscle mass and sex drive which, as the author who is a male in his sixties experienced, can be maintained at levels corresponding to those of someone in his early forties or even thirties. However instead of supplements inducing one's own body secretion of gender specific hormones, existing hormone replacement or supplementation therapies can also possibly be used, but due to the known possibility of serious complications including oncological it's not our preferred approach, even though a variety of effective cancer prevention supplements are available. The author used the treatment of this invention on himself since 2004 and observed its results. Besides the initial biological age reduction and subsequent drastic aging slowdown the benefits observed included; mental acuity and memory remaining unchanged, 3cm increase in height over 16 years and eliminating any progression of dental caries or formation of new caries spots and preventing the onset of periodontitis which normally would be expected as a person enters their sixties and commonly much earlier.

Fluoride supplementation has long been used for caries prevention and is well known in the art, but it's effectiveness is limited whereas fluoride is toxic and, in addition to thyroid and endocrine dysfunction, produces significant permanent IQ reduction in children (as has been established by a plurality of readily findable studies) which caused substantial population's IQ reduction in countries that practiced fluoridation of water; also largely negating, in the developed countries that practiced water fluoridation, the well known Flynn Effect of steady increase in National IQ Averages over the decades. Said decline in population's cognitive abilities in turn produced major adverse effects on the countries where fluoridation was practiced. In many countries and localities today fluoridation has been discontinued and a new solution is urgently needed.

The supplements and vitamins used in the proposed treatment are very inexpensive (in US melatonin and chromium picolinate doses per person per day cost a few pennies) and are readily available without a prescription in most countries. In countries where there is no population decline, they can be used to rejuvenate the most valuable society members such as distinguished scientists and accomplished members of various professions or selected by different criteria such as IQ above certain level or having a particular ability, such as for example mathematical ability or organizational ability. Alternatively, especially in countries facing population declines, the entire productive and/or creative parts of population can be treated on a massive scale far postponing their retirement, not only preventing targeted population's numerical decline, but producing a substantial surge in population numbers due to postponed mortality resulting in the swelling of said population numbers and enabling them to have more children at a later age. Keeping the population biologically younger will make it much healthier; that and the prevention of cognitive decline makes it possible to achieve highly substantial economic benefits. Largely eliminating the aforementioned dental diseases will also produce very substantial savings for any country.

As the maintenance of body's physiological condition corresponding to substantially younger age despite the chronological progression of age, would be threatened by common major diseases such as diabetes, heart disease and cancer; it would be preferable to use the known, readily available supplements preventing or suppressing such diseases, despite the common misconception in the public and even some doctors that they do not exist. For example diabetes type 2 is prevented and in the majority of cases is cured within 2 to 3 weeks by taking of chromium supplements 400-500 mcg per day (5) and also is prevented and usually responds to the treatment by alpha lipoic acid of 300 mg per day (6) more or less and thus the incidence of it would likely be much reduced if the treatment of this invention comprising the taking of these supplements is used. Most effective supplement today for cancer prevention is quercetin 400-500 mg/day, as well as vitamin B1 100 mg/day or more, rutin and apigenin. There is a number of effective supplements for prevention of atherosclerosis, hypertension and heart disease of which the most promising appears to be nattokinase, at least 200 mg/day. Finally regarding the increasingly frequent epidemics of viral diseases the most potent supplement for protection against most viral diseases is zink 50 mg/day which can be strengthened even further by taking it with quercetin in the above mentioned
dosage.

### 3. Objects and Advantages

One object of the present invention is to provide a supplement based regimen for much reducing the biological age and afterwards drastically slowing the aging of the whole body.

Another object of the present invention is largely eliminating diabetes type 2 in the treated population.
Another object of the present invention is drastic prolongation of the treated persons healthspan and lifespan

Another object of the present invention is to provide a supplement based regimen assuring the maintenance or limited decline of body's muscle mass despite the chronological progression of age even without exercise beyond the normal daily activities.

Another object of the present invention is to provide a supplement based regimen assuring the maintenance of a person's cognitive ability despite the chronological progression of age. Another object of the present invention is to provide a supplement based regimen to enable persons to grow taller in the chronological middle age and beyond.

Another object of the present invention is to provide a supplement based regimen to generally prevent the onset and progression of periodontitis and caries.

### 4. Brief Description of the Drawings.

### N.A.

### 5. Description.

The supplement and vitamin regimen of this invention primarily consists of taking melatonin in the usual recommended amounts of 3mg per day more or less. The author based on his experience of taking it continuously for about 20 years identified as optimal (at least for him) the amount of 1.5 mg per day at bedtime finding that amount sufficient (even though he, as a weightlifter, has body mass well above 100 kg), while not producing drowsiness the following day. The rejuvenation effects of melatonin are well known, as it is a recognized geroprotector . Melatonin also makes the immune system stronger and has strong antioxidant and significant anti-cancerogenic properties (4). Another supplement comprising this regimen is preferably chromium picolinate (Cr(Pic)3) or possibly other chromium compounds such as chromium polynicotinate, chromium chloride etc that are to be taken preferably at bedtime together with melatonin. The author used chromium supplementation daily for more than 20 years in the amount of 400 to 500 mcg per day, however as the author discovered in most recent years this therapy can be used in maintenance regime with dosages of chromium supplement 200 mcg/day without any worsening of body's condition compared to that under said higher dosage. Supplementation by chromium compounds, usually intended to improve carbohydrate metabolism, does produce improved glycemic control (5) and also lowers plasma levels of several inflammatory markers; it also produces increased level of Human Growth Hormone (HGH) in serum which is known to promote protein anabolism while reducing fat deposits (see 7). Chromium supplementation also was found to extend the average lifespan for laboratory rats by 87.5% (see 8) when chromium picolinate was used (significantly less for other chromium supplements), but by itself it doesn't produce any visible rejuvenation. A theoretical explanation of the drastic increase in longevity has been proposed (11). However when chromium supplementation is done at about the same time with melatonin supplementation in the evening the rejuvenation effect observed was synergistic, quick and profound - far exceeding what could be accomplished with melatonin supplementation alone. Chromium supplements may have side effects for some people and in that case another supplement long used to improve carbohydrate metabolism, namely alpha-lipoic acid may be used instead. Third component of this regimen comprises orally taken supplements or gels rubbed into skin for maintenance of gender specific hormones at a level within the range corresponding to that of a younger person of the same gender. For men it is vitamin E preferably from natural sources taken in the amount of 400 iu per day more or less, which the author used for about 3 decades observing strongly increased sex drive indicative of high testosterone levels. As a consequence of the rejuvenation treatment in conjunction with high testosterone level there was no substantial decline in muscle mass with age or when not exercising for several months due to injury etc and being able to relatively quickly progress to higher loads and weights when resuming doing strength exercises which continues to this day with no discernible difference for the author who is presently in his late-sixties. For periods of time the author used a costlier mixture of tocotrienols with similar effect, but tocotrienols also have well known strong anti-cancer effects, decrease the incidence of cardio-vascular events and prevent metastases for cancer patients. Here is a research study that confirms the author's observation that vitamin E induces production of higher levels of testosterone in men (see 9) which in turn promotes maintenance or increase of muscle mass. Naturally maintaining higher testosterone levels is also conducive to extending and enhancing men's reproductive ability. For women there are other supplements causing maintenance of women's hormones such as estrogen and progesterone at a level range corresponding to significantly younger age. In author's observation women had the most success with peptide gel products effectively restoring female body's natural hormones production (even long after menopause) such as Zhenoluten^{®} and Peptide Complex PK-10^{®} produced by Scientific Manufacturing Center of Revitalization and Health in St. Petersburg, Russia. After body's hormones production capability is substantially restored, cheaper supplements for enhancing female sex drive may be used which stimulate secretion of the pertinent female hormones - a number of such supplements are commercially available. Another particularly good option is to use 3 mg daily of melatonin to restore female hormone production, which has been shown (12) to restore not only the female hormones, but the menstrual cycle and the ability to get pregnant as well for women who did not complete the menopause; thus the transition to rejuvenation can be done once the hormone levels are restored, by starting to take a chromium supplement in addition to melatonin. As melatonin has a limited rejuvenation effect of its own, by the time chromium supplementation is added some limited rejuvenation will have occurred and if the melatonin dosage is kept at 3 mg the resulting drastic rejuvenation won't be sudden and thus shouldn't be of a concern. Also a known hormone replacement therapy, taking into account the risks and side effects that it entails, is an option that could be used. Alternatively a variety of supplements and/or herbal treatments for boosting the levels of estrogen exist, such as for example red clover and black cohosh. There are supplements, such as Vitamin C for example, that can be used for boosting progesterone levels. The level of testosterone in males or of female hormones most readily manifests itself in the intensity of sex drive which at about the age of sixty, in the author's experience, started to decline necessitating in addition to vitamin E the taking of another supplement to prevent any such decline. There are many testosterone boosters commercially available, but the author, while continuing to use vitamin E, used a well known Chinese herbal supplement Fo Ti to boost testosterone while also preventing the progression of balding and graying of hair. Also to reset author's perceived biological age to 3 to 5 years younger stage a known geroprotector carnosine was added to daily regimen after reaching the age of 60 when the heretofore imperceptibly slow progression of aging became slightly more visible; two 500 mg capsules per day had the desired effect and then for maintenance that was reduced to one capsule per day (taking up to 3 such capsules remains an option) - thus carnosine is deemed to be the fourth, optional part of this treatment regimen. Carnosine's additional benefits are well known: slowing of telomere shortening, it has anti-cancerogenic properties and contributes to preservation of mental clarity in older age. There is a number of available other inexpensive anti-aging supplements to add to daily regimen if that becomes necessary due to aging perhaps again becoming discernible at later chronological ages; they are readily findable as Geroprotectors.

Over the years of using this treatment the author didn't observe any decline in his cognitive abilities and his writing of the present patent application, when the author's chronological age is in late sixties, is evidencing that circumstance, however for people just starting the rejuvenation in late or even mid-fifties the supplements removing mind fogginess and improving memory would be desirable or else these patients might not even be able properly follow the treatment regimen, using fisetin or acetyl carnitine would be advisable. However, as the National Wealth is known to correlate with National IQ Average (13), it makes sense to also include cognitive enhancers, such as for example noopept, to increase the targeted population's IQ average into the treatment of said targeted population to improve their economic performance and by extention the national economic performance. As far as the dental-related improvements are concerned, the author discovered that the expected onset of periodontitis didn't take place; falling out of a deep filling, inadvertently left unfilled, didn't result in tooth infection and then pulpit. Even when a crown fell off leaving exposed the dentine surfaces of a truncated tooth (and was left that way as an experiment in view of the preceding) - that tooth too, didn't become infected, but grew enamel surfaces and didn't cause any complications for years ever since. No new caries spots appeared since the beginning of this supplement treatment. In our experience the most effective chromium supplements were either chromium picolinate or chromium polynicotinate. There were studies performed on rats that established that melatonin in large doses (per unit of body's weight exceeding the doses used by this author by more than 30 times) inhibits the development of caries and periodontitis (10). Alpha-lipoic acid has also been studied for its antimicrobial effect against dental disease-causing oral microorganisms. Regarding dosage and methods of use; the dental cavities may well appear in early teenage years or even before when the body mass children/adolescents of that age may average 50 - 60 kgs so for them the dosage of melatonin and chromium supplements as used by the author can be approximately halved. However as there were reports that melatonin causes delay of puberty, the dental treatment of this invention for pre-pubertal individuals should only be done under medical supervision.

To prevent the unplanned rejuvenation by those unable to support themselves, prison inmates and institutionalized individuals - the melatonin needs to be made available by prescription only. Possibly the melatonin and a chromium compound can be combined in one tablet, possibly optionally also containing carnosine.

There can be many ways and variations to implement the treatment of this invention, but *mutatis mutandis* they are all considered to be within the spirit and scope of this invention.

### 6. Sketches and Diagrams.

### N.A.

### 7. Operation.

The use of supplement and vitamin regimens of this invention was adequately described in the Description section and will not be reiterated here, but is included by way of reference as if fully set forth. However further details and author's experience with the proposed treatment regimen are presented.

Results obtained from this treatment were as follows; drastic rejuvenation occurred within a month and a half to two months if melatonin dosage used was 1.5 mg per day. When melatonin usage was 3 mg per day that extent of rejuvenation was mostly achieved after one week and in the second week maximum possible for this method rejuvenation is achieved. Rejuvenation has been observed combined with drastic weight loss if there was substantial excessive weight *ab initio.* Rejuvenation included elimination of all facial wrinkles and the face acquiring decidedly youthful look, with child-like skin even though the proportions of nose and ears remained corresponding to chronological age. However if the gender specific hormone levels are too low all the rejuvenation achieved may only last for several days and then be lost within a week with evidence of adverse health effects such as baggy eyes (consistent with the breakdown and detritus of newly formed tissues overloading kidneys) and facial swelling, which returns to normal after a few to several months. However, in our experience, such reversal of rejuvenation can be stopped, as soon as it starts by using three 500 mg capsules of carnosine per day, nonetheless gender hormone level deficiency needs to be corrected the sooner the better, as described above. Accordingly in view of this experience the preferred approach is to start the melatonin/chromium treatment for persons in their early to mid-forties, when their hormone levels are normally high enough, as rejuvenation and aging prevention treatment. For individuals where low gender hormone levels are suspected due to low sex drive etc., appropriate blood tests need to be done and rejuvenation treatment not administered if hormone levels are indeed low until hormone levels are rectified. That supplement treatment should continue for the following years in combination with gender hormone level enhancement as needed, as described above. As soon as the aging advancement becomes minimally visible again, likely closer to the age of 60, that treatment needs to be reinforced preferably with carnosine as described. Using alternative treatment; alpha-lipoic acid with melatonin (instead of a chromium supplement and melatonin) doesn't produce rejuvenation as quick and profound as with a chromium supplement, but on the other hand such achieved rejuvenation doesn't reverse if the level of gender specific hormones is too low. Regarding the recommended dosage of alpha-lipoic acid; some manufacturers of it recommend 150 mg/day, other manufacturers recommend one pill of 300 mg and even 600 mg per day. Author used 150 mg/day dosage and was satisfied with results, but noticed that chromium supplements produced substantial blood pressure reduction within minutes after taking, while alpha-lipoic acid supplement didn't have that effect. Author also tried using 450 mg/day dosage of alpha-lipoic acid - no benefits or side effects from increased dosage were observed.

### Clauses

1. A supplement therapy method for the purpose of biological age reduction with subsequent drastic slowdown of aging of the whole body comprising the daily taking of melatonin and a chromium supplement in amounts sufficient to achieve said purpose.
2. A supplement therapy method for the purpose of biological age reduction with subsequent drastic slowdown of aging of the whole body comprising the daily taking of melatonin and alpha-lipoic acid in amounts sufficient to achieve said purpose.
3. The method of Clause 1 further comprising the daily taking of gender-appropriate male or female respectively hormone secretion boosting supplements in the amount sufficient to achieve said purpose.
4. The method of Clause 2 further comprising the daily taking of gender-appropriate male or female respectively hormone secretion boosting supplements in the amount sufficient to achieve said purpose.
5. The method of Clause 1 further comprising the daily administration of gender specific hormone replacement therapy in the amounts sufficient to achieve hormone levels in the blood corresponding to the desired biological age's levels.
6. The method of Clause 2 further comprising the daily administration of gender specific hormone replacement therapy in the amounts sufficient to achieve hormone levels in the blood corresponding to the desired biological age's levels.
7. The method of Clause 1, for the purpose of strengthening the rejuvenation and aging slowdown effects, further comprising the daily taking of at least one additional geroprotector in the amount sufficient to achieve said purpose.
8. The method of Clause 2, for the purpose of strengthening the rejuvenation and aging slowdown effects, further comprising the daily taking of at least one additional geroprotector in the amount sufficient to achieve said purpose.
9. The method of Clause 7 where said additional geroprotector is carnosine.
10. The method of Clause 8 where said additional geroprotector is carnosine.
11. A supplement therapy method for the purpose of prevention of dental caries and periodontitis comprising the daily taking of melatonin and a chromium supplement in amounts sufficient to achieve said purpose.
12. The supplement therapy method of Clause 11 for the purpose of prevention of dental caries and periodontitis wherein said chromium supplement is chromium picolinate supplement in amounts sufficient to achieve said purpose.
13. The method of clause 1, further comprising daily administration of a male or female sex-appropriate sex hormone secretagogue supplement to increase the level of said hormones in doses sufficient to achieve said purpose, prior to the initiation of melatonin and chromium supplementation for rejuvenation.
14. The method of clause 1, further comprising daily administration of melatonin and chromium supplement in doses sufficient to achieve said purpose and contained in a single tablet or capsule.
15. The method of clause 11, further comprising daily administration of melatonin and chromium supplement in doses sufficient to achieve said purpose and contained in a single tablet or capsule.

### 8. References

1. "A terminal metabolite of niacin promotes vascular inflammation and contributes to cardiovascular disease risk" https://www.nature.com/articles/s41591-023-02793-8
2."NAD+ Precursors Nicotinamide Mononucleotide (NMN) and Nicotinamide Riboside (NR): Potential Dietary Contribution to Health" https://www.ncbi.nlm.nih.gov/pmc/articles/PMC10240123/
3."David Sinclair Anti-Aging Supplement Protocol" https://www.nad.com/news/david-sinclairs-2024-anti-aging-supplement-protocol
4."Melatonin as antioxidant, geroprotector and anticarcinogen" https: www.researchgate.net publication 7101877 Melatonin_as antioxidant geroprotector and anticarcinogen
5. "Chromium in the Prevention and Control of Diabetes" https://pubmed.ncbi.nlm.nih.gov/10705100/
6. "Diabetes and Alpha Lipoic Acid" https://www.ncbi.nlm.nih.gov/pmc/articles/PMC3221300/
**7.** "Effects of chromium picolinate supplementation on growth hormone secretion and pituitary mRNA expression in finishing pigs." https://www.thefreelibrary.com/Effects+of+chromium+picolinate+supplementation+on+ growth+hormone...-a0185654022
8. "Longer Life in Rats Linked to Chromium" https://www.latimes.com/archives/la-xpm-1992-10-20-mn-404-story.html
9. "Effect of Vitamin E on Function of Pituitary-Gonadal Axis in Male Rats and Human Subjects" https://www.jstage.jst.go.jp/article/endocrj1954/29/3/29 3 287/ article
10. "Action of Melatonin on Caries Development in Rats" https://journals.sagepub.com/doi/abs/10.1177/00220345760550034401
11. "Longevity Effect of Chromium Picolinate - "Rejuvenation" of Hypothalamic Function?" https://pubmed.ncbi.nlm.nih.gov/7838011/
12. "Effects of melatonin in perimenopausal and menopausal women" Effects of melatonin in perimenopausal and menopausal women: our personal experience - PubMed (nih.gov)
13. " Intelligence and the Wealth or Poverty of Nations" http://www.rlynn.co.uk/uploads/pdfs/Intelligence%20and%20the%20Wealth%20and%20Povert y%20of%20Nations.pdf

## Claims

1. Melatonin in combination with an anti-inflammatory regulator of carbohydrate metabolism for use in reducing biological age and slowing aging in a human subject.

2. The combination for use according to claim 1, wherein the anti-inflammatory regulator of carbohydrate metabolism is a chromium supplement.

3. The combination for use according to claim 1, wherein the anti-inflammatory regulator of carbohydrate metabolism is alpha-lipoic acid.

4. The combination for use according to any of claims 1-3, wherein the treatment further comprises administering a gender-appropriate hormone secretion-boosting supplement in an amount sufficient to achieve hormone levels corresponding to a desired biological age.

5. The combination for use according to any of claims 1-3, wherein the treatment further comprises administering gender-specific hormone replacement therapy in an amount sufficient to achieve hormone levels corresponding to a desired biological age.

6. The combination for use according to any of claims 1-3, wherein the treatment further comprises administering a sex-appropriate hormone secretagogue prior to initiation of melatonin and chromium supplementation.

7. The combination for use according to any of claims 1-3, wherein the treatment further comprises administering at least one additional geroprotector.

8. The combination for use according to claim 7, wherein the additional geroprotector is carnosine.

9. Melatonin in combination with a chromium supplement for use in preventing dental caries and periodontitis.

10. The combination for use according to claim 9, wherein the chromium supplement is chromium picolinate.

11. The combination for use according to any of claims 1-3, wherein melatonin and the chromium supplement are administered together in a single tablet or capsule.

12. The combination for use according to any of claims 9-10, wherein melatonin and the chromium supplement are administered together in a single tablet or capsule.
